# EUROPÄISCHE PATENTSCHRIFT

(11) **EP 2 039 382 B1**
(45) Veröffentlichungstag und Bekanntmachung des Hinweises auf die Patenterteilung: **12.05.2010**
(21) Anmeldenummer: 07017173.1
(22) Anmeldetag: 01.09.2007
(51) Int. Cl.: A61M 5/31, A61M 5/315

(54) **Ampulle mit Dichtung in zwei Kompressionszuständen**
Ampoule with seal in two compression states
Ampoule dotée d'un joint en deux états de compression

(43) Veröffentlichungstag der Anmeldung: 25.03.2009
(73) Patentinhaber: F.HOFFMANN-LA ROCHE AG, 4070 Basel (CH); Roche Diagnostics GmbH, 68305 Mannheim (DE)
(72) Erfinder: Fehlmann, Daniel, 5053 Staffelbach (CH); Horisberger, Ronny, 3424 Oberösch (CH); Zihlmann, Ruedi, 3550 Langnau i.E. (CH)
(74) Vertreter: Poredda, Andreas

(56) Entgegenhaltungen:
- EP-A- 0 248 210
- WO-A-00/71185
- US-A- 3 016 896

## Beschreibung

Die vorliegende Erfindung betrifft eine Ampulle für eine fliessfähige Substanz, insbesondere eine Ampulle für eine medizinische Wirkstofflüssigkeit zur Verabreichung mittels einer Infusions- oder Injektionsvorrichtung, sowie ein Dosiersystem mit einer erfindungsgemässen Ampulle.

Aus der WO 2000/71185 A1 ist eine Medikamentenampulle mit einem Ampullenkörper aus Kunststoff bekannt, bei der ein Kolben zunächst mit einer Spielpassung von dem Ampullenkörper aufgenommen wird und nach der Sterilisierung des Kolbens sowie des Ampulleninnenraums in einen angrenzenden Bereich des Innenraums des Ampullenkörpers verschoben wird, in dem der Kolben gemeinsam mit dem Ampullenkörper eine Dichtung ausbildet, wobei die Dichtung durch eine entgegengesetzte Bewegung des Kolbens wieder aufhebbar ist.

Infusionspumpen nach dem Prinzip der Spritzenpumpe werden für zahlreiche medizinische Anwendungen zur kontinuierlichen Medikamentenversorgung mit einer konstanten oder zeitlich variablen Rate eingesetzt. Ein Beispiel hierfür ist die Therapie von Diabetes Mellitus mittels kontinuierlicher subkutaner Insulininfusion. Hierfür wurden miniaturisierte Infusionspumpen entwickelt, die nach dem Prinzip der Spritzenpumpe arbeiten. Eine solche Pumpe ist z. B. aus der EP 985419 B1 bekannt.

Aus der WO 0160434 A1 ist ein Kolben zum Einsatz in Ampullen für derartige Infusionspumpen bekannt, der einen Grundkörper und ein mit dem Grundkörper stoffschlüssig verbundenes Dichtelement aufweist. Der Grundkörper bestehet dabei z. B. aus einem thermoplastischen Werkstoff, z. B. Polypropylen, und das Dichtelement aus einem thermoplastischen Elastomer, z. B. Santoprene.

Stopfen mit Dichtelementen aus thermoplastischen Elastomeren können jedoch aufgrund ihrer spezifischen Eigenschaften die fluidische Dichtigkeit nach dem Einpressen des Stopfens in den Ampullenkörper nicht über die gesamte geforderte Lagerungsdauer von mehreren Jahren unter wechselnden Klimabedingungen garantieren, da sie sich unter dem vom Ampullenkörper auf sie einwirkenden Druck dauerhaft verformen. Ferner tritt bei der Lagerung einer Ampulle mit eingepresstem Stopfen über längere Zeit hinweg insbesondere bei hoher Lagertemperatur generell auch eine langsame Deformation des Ampullenkörpers unter dem vom Stopfen auf sie einwirkenden Druck (Kriechen) auf, was sich ebenfalls nachteilig auf die Dichtigkeit auswirkt.

Es ist eine Aufgabe der vorliegenden Erfindung, eine Ampulle für eine fliessfähige Substanz bereitzustellen, die diese Nachteile des Standes der Technik überwindet.

Eine derartige Ampulle besitzt einen Ampullenkörper, dessen Innenraum die fliessfähige Substanz aufnimmt. Bei dieser Substanz kann es sich etwa um eine niedervisköse Flüssigkeit, z. B. Insulin oder eine andere medizinische Wirkstoffflüssigkeit, eine hochvisköse Flüssigkeit oder auch eine pulverförmige Substanz handeln. Als Material kommt für den Ampullenkörper bevorzugt Glas oder ein thermoplastischer Kunststoff zum Einsatz, er kann aber auch aus einem anderen geeigneten Material, z. B. rostfreiem Stahl oder Keramik, bestehen.

Die Ampulle umfasst ferner einen Kolben, welcher mindestens ein Dichtelement aufweist und entlang einer Verschieberichtung im Innenraum des Ampullenkörpers verschiebbar ist. Die Verschieberichtung entspricht dabei zugleich der Bewegungsrichtung des Kolbens zur Ausschüttung der Substanz. In mindestens einer am äusseren Umfang des mindestens einen Dichtelements umlaufenden Kontaktzone kontaktiert der Kolben die innere Mantelfläche des Ampullenkörpers und kann durch diese komprimiert werden. Der Kolben kann etwa aus einem thermoplastischen Elastomer oder einem reinen Elastomer gefertigt sein.

Bevorzugt besitzt der Kolben jedoch einen Grundkörper aus einem anderen Material als das Dichtelement, wobei der Grundkörper besonders bevorzugt aus einem thermoplastischen Werkstoff hoher Steifigkeit , z. B. z. B. Polypropylen und das Dichtelement aus einem thermoplastischen Elastomer oder reinen Elastomer geringer Shore-Härte, z. B. Santoprene, gefertigt ist. Hierdurch kann eine für den Kolben vorteilhafte hohe Steifigkeit mit einer hohen Elastizität des Dichtelementes verbunden werden. Das Dichtelement kann z. B. durch einen O-Ring gebildet und in eine entsprechende Nut im Grundkörper montiert werden, oder es können zwei oder mehr O-Ringe verwendet werden. Vorteilhaft ist es jedoch, Grundkörper und Dichtelement durch eine Herstellung als Zweikomponenten-Spritzgussteil stoffschlüssig und/oder formschlüssig zu verbinden. In weiteren Ausführungsformen werden der Grundkörper und das Dichtelement über eine Schnappverbindung verbunden.

Die innere Mantelfläche des Ampullenkörpers weist entlang der Verschieberichtung einen Führungsbereich und einen Dichtungsbereich auf, wobei der Querschnitt des Innenraums in einer Ebene quer zur Verschieberichtung im Führungsbereich so gestaltet ist, dass das mindestens eine Dichtelement einen ersten Kompressionszustand aufweist, wenn sich die Kontaktzone im Führungsbereich befindet und im Dichtungsbereich so gestaltet ist, dass das mindestens eine Dichtelement einen zweiten Kompressionszustand aufweist, wenn sich die Kontaktzone im Dichtungsbereich befindet. Gegenüber dem ersten Kompressionszustand weist das Dichtelement im zweiten Kompressionszustand eine stärkere Kompression auf. Im zweiten Kompressionszustand bildet die innere Mantelfläche gemeinsam mit dem Dichtelement eine fluidische Dichtung. Der Führungsbereich ist bevorzugt so ausgelegt, dass die innere Mantelfläche das Dichtelement nicht oder nur in so geringem Masse komprimiert, dass das Dichtelement nicht plastisch komprimiert wird, wenn sich die Kontaktzone im Führungsbereich befindet. (Im Gegensatz zu einer elastischen Deformation bleibt eine plastische Deformation auch nach Ende einer Krafteinwirkung dauerhaft erhalten). Eine möglicherweise vorhandene Dichtwirkung gegenüber der fliessfähigen Substanz ist dabei von untergeordneter Bedeutung.

Als Mass für die Deformation des Dichtelementes dient seine Verpressung, welche die prozentuale Kompression des Dichtelementes bezogen auf seine belastungsfreie Ausdehnung angibt. Für die bevorzugt für das Dichtelement eingesetzten thermoplastischen Elastomere sollte diese im Dichtungsbereich nicht grösser als 25% sein und besonders bevorzugt deutlich darunter liegen. Bei einer exemplarischen Auslegung einer erfindungsgemässen Ampulle mit kreiszylindrischem Querschnitt des Ampullenkörpers beträgt der Innendurchmesser des Ampullenkörpers im Dichtungsbereich 6.5 mm und der belastungsfreie Durchmesser des Dichtelementes 6.9 mm. Hieraus ergibt sich eine Durchmesserreduktion des Dichtelementes aufgrund seiner Kompression um 6.9 mm -6.5 mm = 0.4 mm und eine Kompression des Dichtelementes um 0.4 mm/6.9 mm -0.058, entsprechend 5.8%.

Wurde die Konzaktzone zwischen dem Dichtelement und der inneren Mantelfläche des Ampullenkörpers einmal vom Führungsbereich in den Dichtungsbereich hinein verschoben und damit eine fluidische Dichtung gebildet, ist eine Wiederaufhebung der Dichtung nicht möglich. Hierzu ist eine zweite Sperrvorrichtung vorgesehen.

Die zweite Sperrvorrichtung kann mindestens einen kolbenseitigen Anschlag, eine Gleitfläche und einen zweiten ampullenk8rperseitigen Anschlag umfassen. Bevorzugt weist die innere Mantelfläche des Ampullenkörpers gemeinsam mit dem mindestens einen Dichtelement keine Dichtwirkung gegenüber einem Medium mit sterilisierender Wirkung und/oder einer Strahlung mit sterilisierender Wirkung auf, wenn sich das mindestens eine Dichtelement im ersten Kompressionszustand befindet. Hierdurch kann zur Sterilisierung des Innenraums des Ampullenkörpers ein Medium mit sterilisierender Wirkung und/oder Strahlung mit sterilisierender Wirkung in den Innenraum des Ampullenkörpers eindringen, wenn sich das mindestens eine Dichtelement im ersten Kompressionszustand befindet. Bevorzugt weist der Ampullenkörper ferner einen Übergangsbereich auf, der den Innenraum des Ampullenkörpers im Führungsbereich stetig, also ohne Sprünge oder Kanten, mit dem Innenraum des Ampullenkörpers im Dichtungsbereich verbindet.

Aus Sicherheitsgründen sowie zur Verbesserung der Führung des Kolbens im Ampullenkörper kann ferner das Dichtelement so gestaltet werden, dass es mit der inneren Mantelfläche des Ampullenkörpers zwei oder mehr Kontaktzonen umfasst und/oder es können zwei oder mehr Dichtelemente vorgesehen sein.

Im einer bevorzugten Ausführungsform umfasst der Kolben mindestens ein gegenüber dem mindestens einen Dichtelement in Richtung einer Öffnung des Ampullenkörpers versetzt, angeordnetes Führungselement welches die innere Mantelfläche des Ampullenkörpers kontaktiert. Hierdurch wird sowohl einem Verkippen des Kolbens als auch durch die zusätzliche Reibung einer ungewollten Verschiebung des Kolbens im Ampullenkörper entgegengewirkt.

Bevorzugt weist eine erfindungsgemässe Ampulle eine Aufziehvorrichtung zur Verschiebung des Kolbens entgegen der Verschieberichtung auf. Diese kann etwa beim Befüllen der Ampulle erforderlich sein, um den ganz in die Ampulle eingeschobenen Kolben zurückzuziehen. In einer bevorzugten Ausführungsform ist die Aufziehvorrichtung in Form einer Kolbenstange ein integraler Bestandteil des Kolbens selbst. In einer anderen bevorzugten Ausführungsform ist die Aufziehvorrichtung, z. B. in Form einer Kolbenstange, lösbar mit dem Kolben koppelbar.

Um eine ruckfreie Bewegung des Kolbens im Innenraum des Ampullenkörpers und geringe Reibungskräfte zu gewährleisten, umfasst eine erfindungsgemässe Ampulle bevorzugt ein visköses Schmiermittel, z. B. Silikonöl, das einen Schmierfilm auf der inneren Mantelfläche des Ampullenkörpers ausbildet. Alternativ oder Zusätzlich ist auch eine Herabsetzung der Reibung durch andere Massnahmen möglich, etwa eine Beschichtung der inneren Mantelfläche des Ampullenkörpers und/oder des Dichtelementes, beispielsweise mit einer Silikon- oder Teflonschicht.

Bevorzugt umfasst der Kolben einer erfindungsgemässen Ampulle eine Koppelvorrichtung zur Kopplung mit einer Vorschubeinheit einer Injektions- oder Infusionsvorrichtung. Die Koppelvorrichtung kann dabei eine Planfläche umfassen, auf welche durch eine von der Vorschubeinheit umfassten Druckplatte eine Druckkraft ausgeübt werden kann. In einer weitern Ausführungsform umfasst die Koppelvorrichtung ein Aussengewinde oder eine Zahnstange, welche mit einer Schlossmutter oder einem Zahnrad einer Vorschubeinheit in Eingriff stehen kann.

Bevorzugt ist der Ampullenkörper mit mindestens einer Auslassöffnung versehen, die sowohl zum Befüllen der Ampulle als auch zur Ausschüttung genutzt wird. Die . Anordnung der mindestens einen Auslassöffnung ist in einer Ausführung koaxial zu einer Symmetrieachse des Ampullenkörpers, wie es von üblichen Spritzen bekannt ist.

In einer weiteren Ausführung ist eine Auslassöffnung quer zur Verschieberichtung angeordnet. Die Auslassöffnung mündet in einer bevorzugten Ausführung in einen Luer-Konus, wie er im medizinischen Bereich üblich ist. In einer anderen weiteren Ausführung ist die Auslassöffnung mit einem Septum abgeschlossen, welches zur Entlüftung, zur Befüllung und zur Entleerung des Ampulleninnenraums von einer Hohlkanüle durchstochen werden kann.

Wenn sich die Kontaktzone zwischen der inneren Mantelfläche der Ampulle und dem Dichtelement im Führungsbereich befindet, ist aufgrund der bevorzugt geringen Kompression des Dichtelementes der Sitz des Kolbens im Ampullenkörper lose. Um in diesem Zustand ein ungewolltes Entfernen des Kolbens aus dem Ampullenkörper zu verhindern, ist bevorzugt eine erste Sperrvorrichtung vorgesehen. Die erste Sperrvorrichtung kann mindestens einen kolbenseitigen Anschlag, eine Gleitfläche und einen ersten ampullenkörperseitigen Anschlag umfassen.

Weist eine erfindungsgemässe Ampulle eine erste Sperrvorrichtung auf, erweist es sich als vorteilhaft, die erste und die zweite Sperrvorrichtung zueinander analog auszuführen. Insbesondere kann der mindestens eine kolbenseitige Anschlag der ersten Sperrvorrichtung identisch sein mit dem mindestens einen kolbenseitigen Anschlag der zweiten Sperrvorrichtung. Zusätzlich kann die mindestens eine Gleitfläche der ersten Sperrvorrichtung identisch sein mit der mindestens einen Gleitfläche der zweiten Sperrvorrichtung.

Ein für eine erste Sperrvorrichtung und eine zweite Sperrvorrichtung identischer kolbenseitiger Anschlag sowie ein für eine erste Sperrvorrichtung und eine zweite Sperrvorrichtung gemeinsame Gleitfläche können besonders vorteilhaft als Sperrklinke ausgebildet sein, welche an einem vom Kolben umfassten und zu einer offenen Stirnfläche des Ampullenkörpers weisenden und radial federnden Federarm angeordnet ist. Ferner weist die Wandung des Ampullenkörper in dieser Ausführung mindestens einen ersten Ausschnitt und mindestens einen zum ersten Ausschnitt in Verschieberichtung R versetzten zweiten Ausschnitt auf, wobei der erste ampullenkörperseitige Anschlag durch die Wandung des mindestens einen ersten Ausschnitts gebildet wird und der zweite ampullenkörperseitige Anschlag durch die Wandung des mindestens einen zweiten Ausschnitts gebildet wird. Die mindestens eine Sperrklinke kann dabei im entlasteten Zustand des Federarmes in den mindestens einen ersten Ausschnitt sowie alternativ in den mindestens einen zweiten Ausschnitt hineinragen.

Ferner kann der mindestens eine Federarm so gestaltet sein, daß ihn die innere Mantelfläche des Ampullenkörpers so deformieren kann, dass die mindestens eine Sperrklinke federnd gegen die innere Mantelfläche der Ampulle drückt. Dies kann die Führung des Kolbens im Innenraum des Ampullenkörpers verbessern sowie aufgrund der Andruckkraft einer ungewollten Verschiebung des Kolbens entgegenwirken.

Der Innenraum des Ampullenkörpers kann senkrecht zur Verschieberichtung des Kolbens verschiedene Querschnittsformen aufweisen, in bevorzugten Ausführungsformen ist die Querschnittsform kreisförmig. In anderen Ausfuhrungsformen weist er einen von der Kreisform abweichenden Querschnitt auf, insbesondere weist er in einer weiteren bevorzugten Ausführungsform eine elliptische Form auf. In einer weiteren, ebenfalls bevorzugten Ausführungsform weist der Innenraum des Ampullenkörpers mindestens ein Paar einander gegenüberliegender, paralleler Begrenzungsflächen auf. Diese können über gekrümmte, z. B. halbzylindrische, Mantelflächen verbunden sein. Eine von der Zylinderform abweichende Form des Ampullenkörpers kann besonders beim einsatz in Injektions- oder Infusionssystemen eine vorteilhafte Raumausnutzung ermöglichen.

Die Bereitstellung einer erfindungsgemässen Ampulle erfolgt bevorzugt in einer sterilen Verpackung, wobei sich das mindestens eine Dichtelement im ersten Kompressionszustand befindet. Bevorzugt wird dabei ein Entfernen des Kolbens aus dem Ampullenkörper durch eine erste Sperrvorrichtung verhindert. Vor der Befüllung der Ampulle wird der Kolben in der Verschieberichtung verschoben, wodurch die Kontaktzone zwischen dem mindestens einen Dichtelement und der inneren Mantelfläche der Ampulle vom Führungsbereich über den bevorzugt vorhandenen Übergangsbereich in den Dichtungsbereich verschoben wird, wobei das mindestens eine Dichtelement in den zweiten Kompressionszustand überführt wird. Durch die anschliessende weitere Bewegung des Kolbens in der Verschieberichtung wird der Ampulleninnenraum entlüftet. Zugleich wird durch die Bewegung des Kolbens ein im Innenraum des Ampullenkörpers bevorzugt vorhandenes visköses Schmiermittel gleichmässig auf der inneren Mantelfläche des Ampullenkörpers verteilt. Sofern die mindestens eine Auslassöffnung mit einem Septum abgeschlossen ist, wird dieses vor der Bewegung des Kolbens mit einer Hohlkanüle durchstochen.

Im Anschluss an die Entlüftung erfolgt die Befüllung mit der Wirkstoffflüssigkeit, welche bevorzugt durch die mindestens eine Auslassöffnung erfolgt, die hierbei als Befüllöffnung genutzt wird. Sofern die mindestens eine Auslassöffnung mit einem Septum abgeschlossen ist, wird dieses mit der bereits zur Entlüftung genutzten oder mit einer weiteren Hohlkanüle durchstochen. Das Befüllen erfolgt bevorzugt durch Zurückziehen des Kolbens entgegen der Verschieberichtung mit Hilfe einer bevorzugt vorhandenen Aufziehvorrichtung, wodurch im zuvor entlüfteten Innenraum des Ampullenkörpers zwischen Auslassöffnung und Kolben ein Unterdruck entsteht, durch welchen die Wirkstofflüssigkeit in den Innenraum des Ampullenkörpers gesaugt wird. Alternativ ist es auch möglich, die Wirkstoffflüssigkeit durch die Auslassöffnung mittels Überdruck in den Innenraum des Ampullenkörpers hineinzudrücken, wobei die Wirkstoffflüssigkeit den Kolben entgegen der Verschieberichtung verschiebt.

Ein zweiter Aspekt der Erfindung betrifft eine Ampulle für eine fliessfähige Substanz, die mindestens eine erste erfindungsgemässe Teilampulle und eine zweite erfindungsgemässe Teilampulle umfasst. Dies ermöglicht eine insgesamt flache Ausführung einer Ampulle grösseren Inhalts unter Beibehaltung eines aus Gründen der Steifigkeit vorteilhaften kleinen Innenquerschnitts der einzelnen Teilampullen. In bevorzugten Ausführungsformen sind Teilampullen gleichartig und weisen insbesondere einen gleichartigen Innenraum auf. Je nach den Platzverhältnissen, z. B. in einer Injektions- oder Infusionsvorrichtung, können die einzelnen Ampullen aber auch unterschiedliche Querschnitte aufweisen. Bevorzugt sind die Teilampullen zu einander so angeordnet, dass sie eine gemeinsame Verschieberichtung aufweisen. Besonders bevorzugt sind in diesem Fall die Kolben der Teilampullen miteinander verbunden, wobei das Verbinddungsstück integral mit den Kolben in einem Teil, z. B. in einem Spritzgussteil, zusammengefasst sein kann. In anderen Ausführungen können die Verschieberichtungen aber auch einen Winkel zu einander aufweisen. Insbesondere können die Einzelampullen auch so zu einander angeordnet sein, dass ihre Verschieberichtungen einander entgegengesetzt sind. Bevorzugt weisen die mindestens zwei Teilampullen eine gemeinsame Auslassöffnung auf. Es können aber auch mehrere Auslassöffnungen, z. B. eine Auslassöffnung je Teilampulle, vorgesehen werden.

Ein weiterer Aspekt der vorliegenden Erfindung betrifft ein Dosiersystem für eine fliessfähige Substanz, insbesondere zur Infusion von Insulin oder anderen medizinischen Wirkstoffen. Ein solches System umfasst eine erfindungsgemässe Ampulle sowie eine Injektions- oder Infusionsvorrichtung, welche mit der Ampulle operativ gekoppelt ist. Insbesondere kann die Injektions- oder Infusionsvorrichtung die Ampulle ganz oder teilweise aufnehmen.

Bevorzugt umfasst das Dosiersystem ferner einen Adapter, welcher lösbar mit der Injektions- oder Infusionsvorrichtung koppelbar ist sowie eine Injektions- oder Infusionskanüle, wobei der Adapter die Auslassöffnung der Ampulle fluidisch mit der Injektions- oder Infusionskanüle koppelt und einen Anschlag zur Festlegung der Ampulle in der Verschieberichtung bildet. Dabei kann sich die Injektions- oder Infusionskanüle unmittelbar an den Adapter anschliessend oder so kann über einen Katheter an den Adapter gekoppelt sein. In einer besonders bevorzugten Ausführungsform umfasst die Auslassöffnung der Ampulle ein Septum, welches von einer vom Adapter umfassten Kanüle durchstochen wird.

Bevorzugt erfolgt die Kopplung des Adapters an die Injektions- oder Infusionsvorrichtung durch eine Relativbewegung zwischen Adapter und Injektions- oder Infusionsvorrichtung quer zur Verschieberichtung, wobei die Relativbewegung zugleich die Auslassöffnung der Ampulle fluidisch mit dem Adapter koppelt.

Der Adapter kann ferner eine Überwachiingsvorrichtung zur Überwachung der Dosierung, etwa in Form eines Drucksensors, eines Druckschalters oder eines Strömungssensors umfassen.

Bevorzugt umfasst die Injektions- oder Infusionsvorrichtung eine Vorschubeinheit zur kontrollierten Verschiebung des Kolbens in der Verschieberichtung.

Insbesondere kann die Vorschubeinheit eine Kolbenstange umfassen, welche durch eine der Vorschubeinheit zugewandten Öffnung der Ampulle in die Ampulle einfahren und so den Kolben kontrolliert innerhalb der Ampulle verschieben kann. Alternativ kann auch der Kolben eine Gewindestange oder eine Zahnstange umfassen, welche mit einer von der Vorschubeinheit umfassten Schlossmutter oder einem Zahnrad in Eingriff stehen kann.

Nachfolgend wird die Erfindung anhand von Ausführungsbeispielen erläutert. Es zeigen:
- Figur 1: Ansicht einer erfindungsgemässen Ampulle entsprechend einem ersten Ausführungsbeispiel;
- Figur 2: Längsschnitt einer Ampulle gemäss Figur 1, wobei sich das Dichtelement im ersten Kompressionszustand befindet;
- Figur 3: Detailansicht der Kontaktzone von Dichtelement und innerer Mantelfläche des in Figur 2 dargestellten Längsschnittes;
- Figur 4: Detailansicht einer ersten und zweiten Sperrvorrichtung des in Figur 2 dargestellten Längsschnittes..
- Figur 5: Längsschnitt einer Ampulle gemäss Figur 1, wobei sich das Dichtelement im zweiten Kompressionszustand befindet;
- Figur 6: Isometrische Ansicht einer erfindungsgemässen Ampulle entsprechend einem zweiten Ausführungsbeispiel;
- Figur 7: Längsschnitt einer Ampulle gemäss Figur 6, wobei sich das Dichtelement im ersten Kompressionszustand befindet;
- Figur 8: Querschnitt des Ampullenkörpers im Dichtungsbereich einer Ampulle gemäss Figur 6;
- Figur 9: Isometrische Ansicht einer erfindungsgemässen Ampulle bestehend aus einer ersten Teilampulle und einer zweiten Teilampulle;
- Figur 10: Längsschnitt einer Ampulle gemäss Figur 9, wobei sich die Dichtelemente der Teilampullen im ersten Kompressionszustand befinden;
- Figur 11: Isometrische Ansicht eines Dosiersystems mit einer Ampulle entsprechend Figur 6 bis Figur 8 und einem Injektions- oder Infusionssystem.

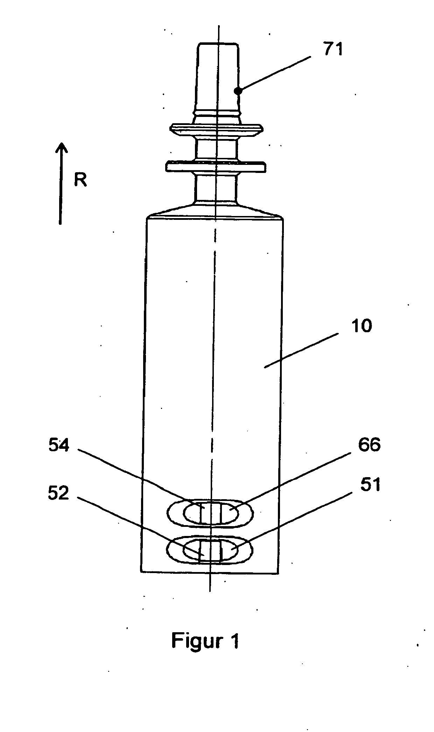

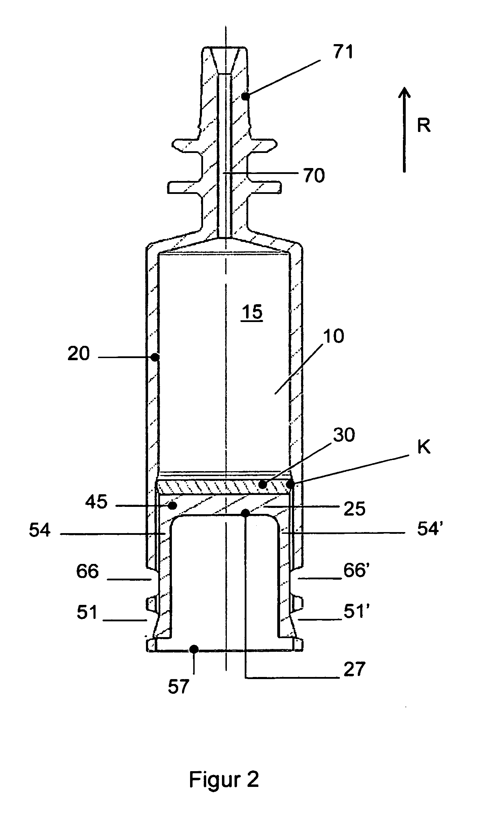

Figur 1 zeigt eine erfindungsgemässe Ampulle entsprechend einem ersten Ausführungsbeispiel und Figur 2 einen Längsschnitt der Ampulle. Der zylindrische Ampullenkörper 10 besteht aus einem thermoplastischen Spritzgussteil, dessen Innenraum 15 zur Aufnahme von Insulin als fliessfähiger Substanz vorgesehen ist. Der Kolben 25 ist ein Zweikomponenten-Spritzgussteil, bestehend aus einem thermoplastischen Grundkörper 45 und einem auf den Grundkörper 45 aufgesetzten Dichtelement 30, welches mit der innere Mantelfläche 20 der Ampulle in einer Kontaktzone K in Kontakt steht. Die zur Öffnung 57 des Ampullenkörpers 10 weisende Stirnfläche des Grundkörpers 45 ist als Planfläche 27 ausgeführt, durch welche eine Druckkraft auf den Kolben 25 übertragen werden kann, woraus eine Verschiebung des Kolbens in einer Verschieberichtung R resultiert. Die Verschieberichtung R entspricht dabei der Längsachse der Ampulle.

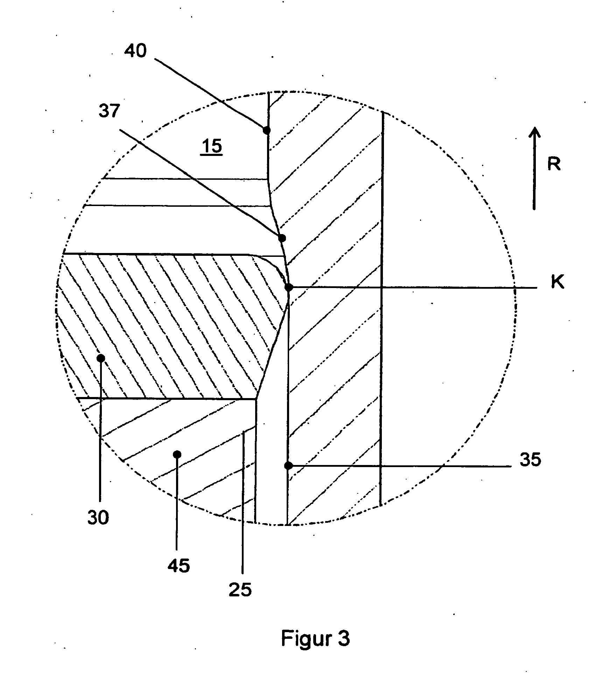

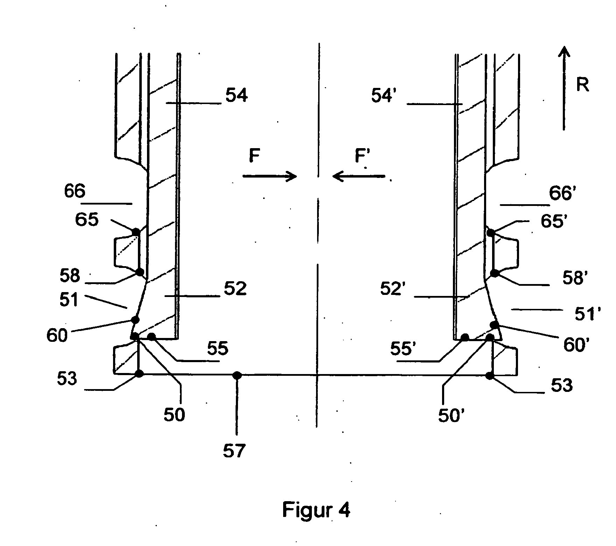

Entsprechend der Darstellung von Figur 2 kontaktiert das Dichtelement 30 die innere Mantelfläche 20 des Ampullenkörpers 10 im Führungsbereich 35. Figur 3 zeigt den Übergang zwischen Führungsbereich 35 und Dichtungsbereich 40 (Detail A in Figur 2). Um ein Entfernen des Kolben 25 aus dem Ampullenkörper 10 zu verhindern, ist eine erste Sperrvorrichtung vorgesehen, die in der Detailansicht Figur 4 dargestellt ist.

Hierzu besitzt der Ampullenkörper 10 an seinem Umfang erste Ausschnitte 51, 51', deren zur Öffnung 57 des Ampullenkörpers weisende kanten erste ampullenkörperseitige Anschläge 50, 50' bilden. Der Kolben 25 weist senkrecht von ihm abstehende, radial elastische Federarme 54, 54' auf, welche in Sperrklinken 52, 52' enden. Die Sperrklinken 52, 52' umfassen jeweils einen kolbenseitigen Anschlag 55, 55' und eine Gleitfläche 60, 60'. Zur Montage wird der Kolben 25 von der Öffnung 57 aus in Verschieberichtung R in den Ampullenkörper 10 eingeführt. Dabei federn die Federarme 54, 54' aus ihrer entspannten Ruhestellung in Federrichtung F, F' radial zur Ampullenachse hin und die Gleitflächen 60, 60' gleiten über die Kante 53 der inneren Mantelfläche des Ampullenkörpers. Sobald bei der weiteren Verschiebung des Kolbens 25 die Sperrklinken 52, 52' in den Bereich der ersten Ausschnitte 51, 51' gelangen, bewegen sich die Sperrklinken 52, 52' mit einer Bewegung entgegen Federrichtung F, F' in ihre entspannte Ruhestellung zurück, so dass sie in die ersten ampullenktirperseitigen Ausschnitte 51, 51' hineinragen. Bei einer möglichen Bewegung des Kolbens 25 entgegen der Verschieberichtung R kommen die kolbenseitigen Anschläge 55, 55' mit den ersten ampullenkörperseitigen Anschlägen 50, 50' in Kontakt, wodurch eine weitere Bewegung des Kolbens 25 entgegen der Verschieberichtung R in Richtung von Öffnung 57 verhindert wird. Eine weiter Verschiebung des Kolbens 25 in Verschieberichtung R ist dagegen möglich, wobei die Gleitflächen 60,60 über die den ersten ampullenkörperseitigen Anschlägen 50, 50' gegenüberliegende Kanten 58, 58' der ersten Ausschnitte 51 gleiten und dabei in Federrichtung F, F' federn.

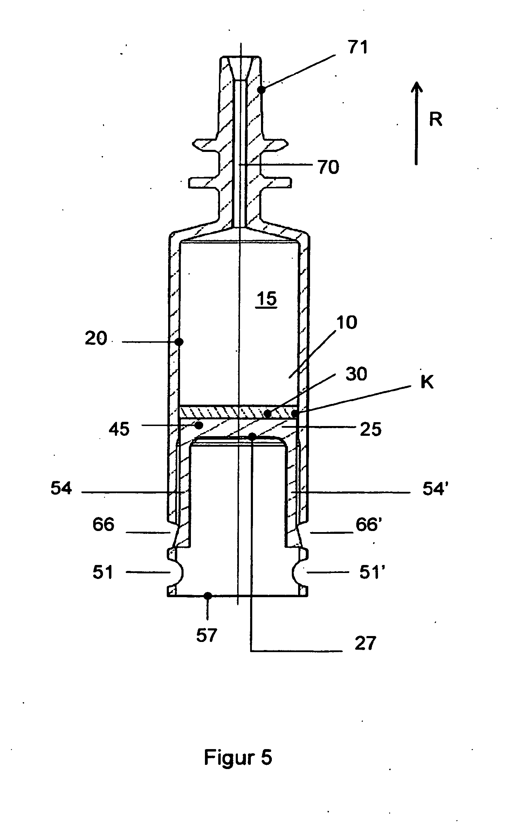

Eine weitere Bewegung des Kolbens 25 in Verschieberichtung R verschiebt die Kontaktzone K vom Führungsbereich 35 in den Dichtungsbereich 40, in dem der Innenraum 15 des Ampullenkörpers 10 einen geringeren lichten Durchmesser aufweist. Hierdurch stellt sich der zweite Kompressionszustand des Dichtelementes 25 ein.Figur 5 zeigt einen zu Figur 1 korrespondierenden Schnitt der Ampulle, wobei sich das Dichtelement 30 im zweiten Kompressionszustand befindet. Um ein Klemmen oder sogar eine mögliche Beschädigung des Dichtelementes 30 bei Verschieben der Kontaktzone K vom Führungsbereich 35 in den Dichtungsbereich 40 zu verhindem, sind beide Bereiche durch einen stetigen Übergangsbereich 37 verbunden. Bei einem zylindrischen Ampullenquerschnitt kann die innere Mantelfläche 20 des Ampullenkörpers 10 im Übergangsbereich 37 im einfachsten Fall durch die Mantelfläche eines zylindrischen Kegelstumpfes gebildet werden, dessen Durchmesser sich vom Innendurchmesser des Ampulleninnenraums 15 im Führungsbereich 35 zum Innendurchmesser des Ampulleninnenraums 15 im Dichtungsbereich 40 verjüngt. Günstiger ist jedoch eine Kontur, im Übergangsbereich 37, welche den Führungsbereich 35 glatt, d. h. ohne Kanten, mit dem Dichtungsbereich 40 verbindet.

Eine Verschiebung der Kontaktzone K entgegen der Verschieberichtung R aus dem Dichtungsbereich 40 heraus in den Führungsbereich 30 würde einen Verlust der Dichtigkeit bedeuten und soll daher verhindert werden. Hierfür ist eine zweite Sperrvorrichtung vorgesehen, deren Funktionsweise analog zur ersten Sperrvorrichtung vorgesehen, mit zweiten Ausschnitten 66, 66' und zweiten ampullenkörperseitigen Anschlägen 65, 65'. Die zweiten Ausschnitte 66, 66' sind dabei gegenüber dem ersten Ausschnitt 51, 51' in der Verschieberichtung R versetzt. Die Federarme 54, 54' mit den Sperrklinken 52, 52' werden sowohl für die erste Sperrvorrichtung als auch für die zweite Sperrvorrichtung genutzt, so dass die kolbenseitigen Anschläge 55, 55' und die Gleitflächen 60, 60' für beide Sperrvorrichtungen identisch sind.

Alternativ sind für die Sperrvorrichtungen auch andere Konstruktionen möglich, z. B. mit zwei in Verschieberichtung R zueinander versetzt auf der inneren Mantelfläche 20 des Ampullenkörpers 10 angeordneten Paaren von Sperrklinken und jeweils einem Ausschnitt in den radial federnden Federarmen 54, 54'.

Bei einer weiteren Bewegung des Kolbens in Verschieberichtung R drücken die Sperrklinken 52, 52' aufgrund der Federkraft der Federarme 54, 54' federnd gegen die innere Mantelfläche 15 des Ampullenkörpers 10. Dies stellt zusätzlich zur Reibung zwischen Dichtelement 30 und innerer Mantelfläche 20 im zweiten Kompressionszustand des Dichtelementes 30 einen Schutz gegen eine ungewollte Verschiebung des Kolbens 25 im Innenraum 15 des Ampullenkörpers 10 dar. Eine Verschiebung des Kolbens 25 resultiert in einer Gleitreibungskraft zwischen der inneren Mantelfläche 20 und den Sperrklingen 52, 52' in einer der Verschiebung entgegengesetzten Richtung. Zugleich wirkt der Kontakt zwischen der inneren Mantelfläche 15 und den Sperrklinken 52, 52' einer Verkippung des Kolbens 25 entgegen.

Zur Sicherstellung einer symmetrischen Krafteinleitung in den Kolben 25 sind die erste Sperrvorrichtung sowie die zweite Sperrvorrichtung jeweils doppelt und auf dem Ampullenumfäng um 180° zueinander versetzt vorhanden. Ebenso wäre z. B. auch eine dreifache Anordnung mit 120°-Winkeln oder anderes möglich. Grundsätzlich wäre auch eine einfache Ausführung der Sperrvorrichtungen möglich.

Der Innenraum 15 des Ampullenkörpers mündet in eine Auslassöffnung 70, mit einem Luer-Konus 71 zur Kopplung an eine übliche Kanüle oder einen Katheter.

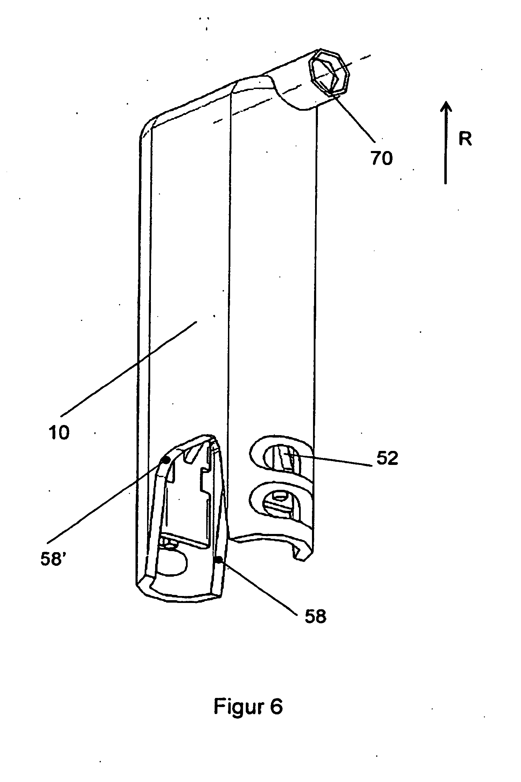

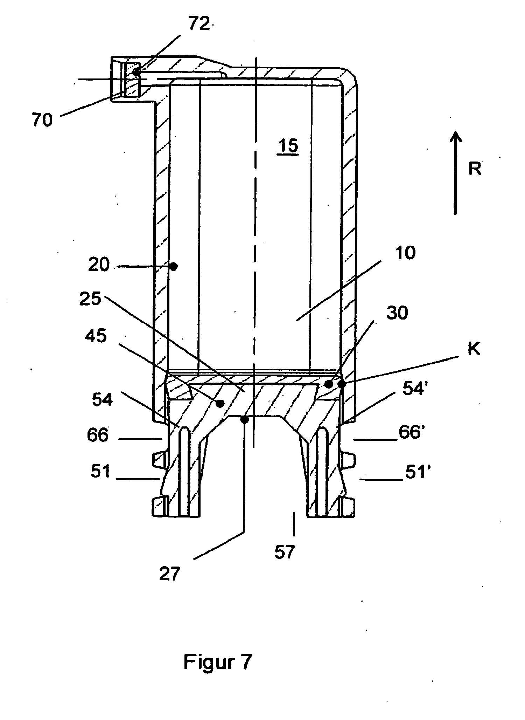

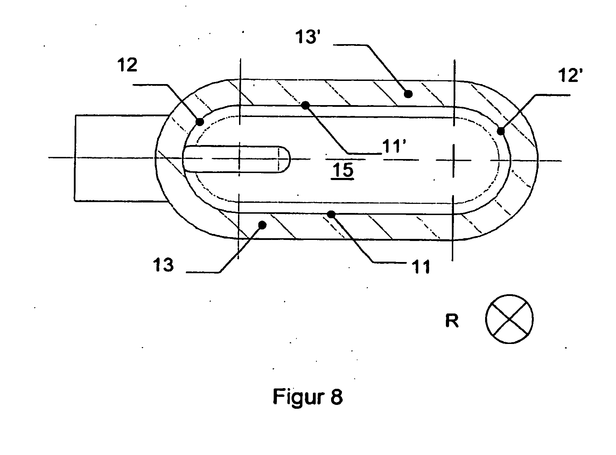

Figur 6 zeigt eine zweite bevorzugte Ausführungsform einer erfindungsgemässen Ampulle, Figur 7 einen Längsschnitt der Ampulle, wobei sich das Dichtelement 30 im ersten Kompressionszustand befindet und Figur 8 einen Querschnitt des Ampullenkörpers 10 im Dichtungsbereich 40. Von der in den Figuren 1 bis 5 dargestellten Ausführungsform unterscheidet sich die zweite bevorzugte Ausführungsform insbesondere durch die Form des Ampullenkörpers 10. Der Innenraum 15 des Ampullenkörpers 10 wird dabei durch zwei planparallele Flächen 11, 11' sowie zwei die Flächen 11, 11' verbindende Zylindermantelflächen 12, 12' begrenzt. Hierdurch ergibt sich im Vergleich zu einer zylindrischen Ampulle eine flachere Bauform, was insbesondere beim Einsatz in Injektions- oder Infusionssystemen vorteilhaft ist. Weiterhin ist in dieser Ausführungsform die Auslassöffnung 70 quer zur Verschieberichtung R angeordnet, was im Vergleich zu einer axialen Anordnung zu einer geringeren axialen Abmessung führt. In der Auslassöffnung befindet sich ein fluidisch dichtes und mit einer Hohlkanüle durchstechbares Septum 72.

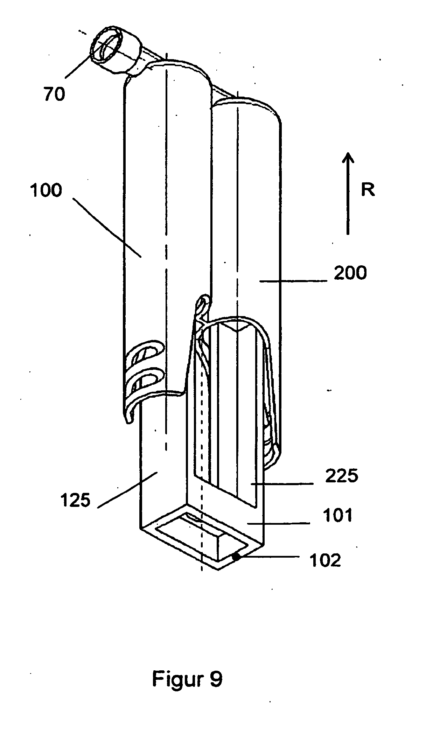

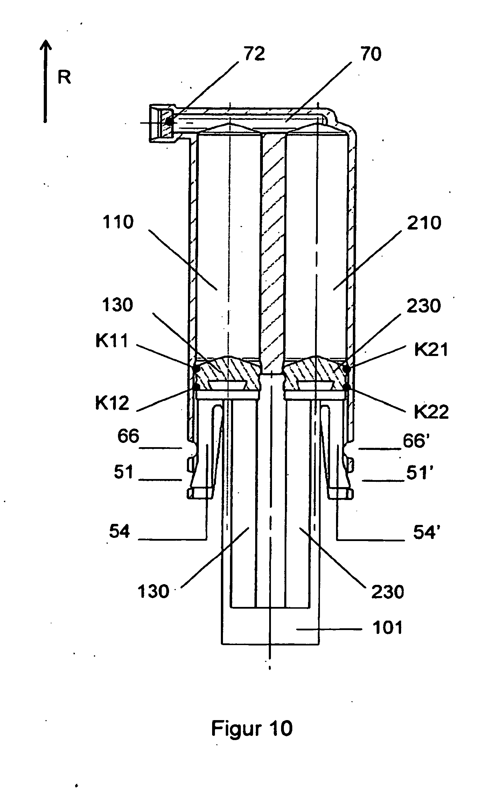

Ausgehend von der Öffnung 57 besitzen die planparallelen Wandungen 13, 13' U-förmige Ausschnitte 58, 58' im Führungsbereich 35. Diese erleichtern ein Greifen des Kolbens 25 selbst oder einer mit dem Kolben 25 lösbar und in Zugrichtung belastbar verbindbarer Aufziehvorrichtung zur Verschiebung des Kolbens 25 entgegen der Verschieberichtung R zur Öffnung 57 hin. Dies ist dann erforderlich, wenn die Ampulle durch die Auslassöffnung 70 mittels Zurückziehen des Kolbens 25 entgegen Verschieberichtung R gefüllt werden soll. Figur 9 zeigt eine Ampulle, welche eine erste erfindungsgemässe Teilampulle (100) und eine zweite erfindungsgemässe Teilampulle (200) umfasst; Figur 10 einen Längsschnitt der Ampulle.

Eine dieser Ansführungsform entsprechende Ampulle umfasst zwei einzelne Teilampullen 100 und 200, die jeweils eine erfindungsgemässe Ampulle darstellen, wobei die Teilampullen 100 und 200 so parallel zu einander angeordnet und über einen Steg verbunden sind, dass sie eine gemeinsame Verschieberichtung Raufweisen. Der Kolben 125 der ersten Teilampulle ist dabei mit dem Kolben 225 der zweiten Teilampulle über ein Verbindungsstück 101 so gekoppelt, dass eine Verschiebung in die gemeinsame Verschieberichtung R oder entgegen der gemeinsamen Verschieberichtung R für Kolben 125 und Kolben 225 gemeinsam erfolgt. Die Stirnfläche 102 von "Verbindungsstück 101 kann zugleich zur Übertragung einer Druckkraft auf die Kolben 125,225 dienen, woraus eine Verschiebung von Kolben 125 und Kolben 225 in Verschieberichtung R resultiert.

Zur Verbesserung der Führung ist das Dichtelement 130 so ausgelegt, dass es in Verschieberichtung R zwei zueinander versetzte Kontaktzonen K 11 und K 12 mit der inneren Mantelfläche 120 des ersten Ampullenkörpers 110 aufweist, und das Dichtelement 230 so ausgeleget; dass es in Verschieberichtung R zwei zueinander versetzte Kontaktzonen K21 und K22 mit der inneren Mantelfläche 220 des zweiten Ampullenkörpers 210 aufweist. Der Ampullenkörper 110 und der Ampullenkörper 210 weisen eine gemeinsame Auslassöffnung 70 quer zur Verschieberichtung R mit einem Septum 72 auf.

Die Dichtelemente 130 und 230 sind mit den Grundkörpern 145 und 245 der Kolben 110 und 210 sowie dem Verbindungsstück 101 als integrales Zweikomponenten-Spritzgussteil ausgeführt.

Aufbau und Funktionsweisen der ersten Sperrvorrichtung und der zweiten Sperrvorrichtung entsprechen der ersten und zweiten bevorzugten Ausführungsform. Die Grundkörper 145 und 245 dienen gemeinsam mit dem Verbindungsstück 101 zugleich als Aufziehvorrichtung für eine Verschiebung der Kolben 125 und 225 entgegen der Verschieberichtung R.

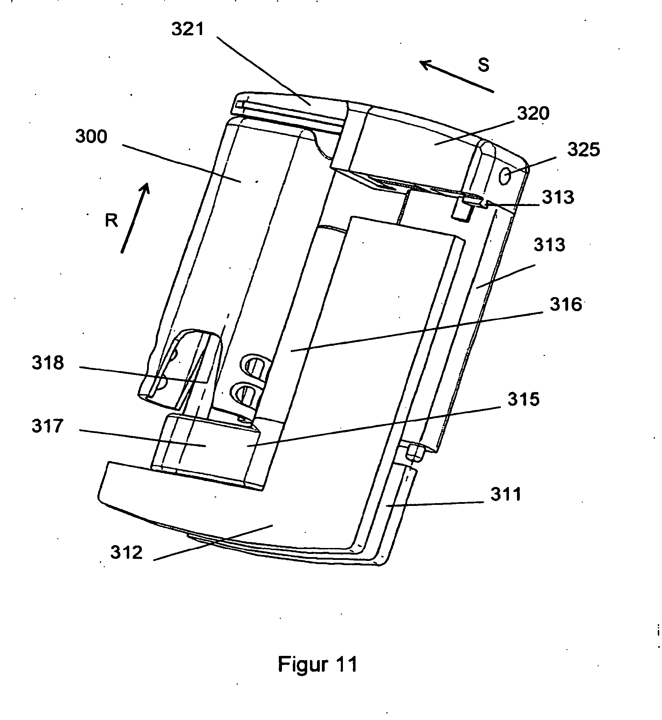

Figur 11 zeigt ein Dosiersystem für insulin oder andere Medikamente, welches eine der zweiten bevorzugten Ausführungsform entsprechende Ampulle 300 sowie ein Infusionssystem umfasst, welches die Ampulle 300 ganz oder teilweise aufnimmt. Das Infusionssystem umfasst eine Vorschubeinheit 315 in Form eines Spindelantriebs mit einem Gleichstrommotor oder Schrittmotor 316, einem Reduktions- und Umlenkgetriebe 317 und eine Kolbenstange 318, Die Kolbenstange 318 ist zur Ausschüttung von Insulin in Verschieberichtung R aus dem Reduktions- und Umlenkgetriebe 317 kontrolliert ausfahrbar und übt dabei mittels einer (nicht dargestellten) Druckplatte eine Druckkraft auf die Planfläche 27 des Kolbens 25, aus woraus eine kontrollierte Verschiebung des Kolbens 25 in Verschieberichtung R resultiert. Ferner umfasst die Infusionsvorrichtung 310 eine elektronische Steuerung 311, ein Benutzerinterface 312 sowie eine Energieversorgung 313. Das Benutzerinterface kann ein z. B. alphanumerisches Display (nicht dargestellt) zur Informationsausgabe an den Benutzer sowie eine oder mehrere Tastelemente (nicht dargestellt) zur Informationseingabe durch den Benutzer umfassen.

Ein Adapter 320 koppelt die Auslassöffnung 70 der Ampulle 300 fluidisch mit einer (nicht dargestellten) Injektions- oder Infusionskanüle. Der Adapter 320 umfasst ferner einen Auslegerarm 321, welcher die Stirnfläche 301 der Ampulle 300 überragt und für die Ampulle 300 so einen Anschlag bildet, welcher die Ampulle in Verschieberichtung R fixiert und bei der kontrollierten Verschiebung des Kolbens 25 durch die Vorschubeinheit 315 ein Gegenlager darstellt.

Das Zusammenfügen der Komponenten des dargestellten Dosiersystems vor seiner Inbetriebnahme erfolgt in folgenden Schritten: (I) Die mit Insulin gefüllte Ampulle 300 wird entgegen der Verschieberichtung R in die Infusionsvorrichtung eingeführt. (2) Der Adapter 320 wird durch eine Verschiebung in Richtung S quer zur Verschieberichtung R mit der Infusionsvorrichtung lösbar gekoppelt. Zur Führung des Adapters 320 dient dabei ein am Adapter 320 befestigter Schwalbenschwanz 321 sowie eine (nicht dargestellte) korrespondierende Nut am Infusionssystem. Durch die selbe Bewegung in Richtung S erfolgt auch die fluidische Kontaktierung des Reservoirs. Hierzu umfasst der Adapter 320 eine (in Figur 1 nicht sichtbare) Hohlkanüle, deren Längsachse der Richtung S entspricht und welche das in der Auslassöffnung 70 angeordnete Septum 72 durchsticht. Die Auslassöffnung 325 des Adapters 320 kann entweder in einen mit dem Adapter integralen Infusionskatheter münden oder einen Infusionskatheter über eine fluidische Kupplung konektieren.

Alternativ kann die Auslassöffnung 325 in eine mit dem Adapter integrale oder über fluidische Kupplung konnektierte Infusionskanüle münden.

Der Adapter 320 kann ferner eine Überwachungsvorrichtung zur Überwachung der Dosierung enthalten, insbesondere einen Druck- oder Durchflusssensor, oder er kann eine flexible Druckübertragungsmembran enthalten, die den fluidischen Druck auf einen in der Infusionsvorrichtungf befindlichen Drucksensor überträgt.

## Patentansprüche

1. Ampulle für eine fliessfähige Substanz, umfassend:
a) einen Ampullenkörper (10) mit einem Innenraum (15) zur Aufnahme der fliessfähigen Substanz und mit einer inneren Mantelfläche (20),
b) einen Kolben (25) mit mindestens einem Dichtelement (30), der im Innenraum (15) entlang einer Verschieberichtung (R) verschiebbar angeordnet ist, wobei das mindestens eine Dichtelement (30) mit der inneren Mantelfläche (20) in einer Kontaktzone (K) entlang des äusseren Umfangs des mindestens einen Dichtelementes (30) in Kontakt steht,
wobei die innere Mantelfläche (20) entlang der Verschieberichtung (R) einen Führungsbereich (35) und einen Dichtungsbereich (40) aufweist,
wobei der Querschnitt des Innenraums (15) in einer Ebene quer zur Verschieberichtung (R) im Führungsbereich (35) so gestaltet ist, dass das mindestens eine Dichtelement (30) einen ersten Kompressionszustand aufweist, wenn sich die Kontaktzone (K) im Führungsbereich (35) befindet und im Dichtungsbereich (40) so gestaltet ist, dass das mindestens eine Dichtelement (30) einen zweiten Kompressionszustand aufweist, wenn sich die Kontaktzone (K) im Dichtungsbereich (40) befindet und dass die innere Mantelfläche (20) gemeinsam mit dem mindestens einen Dichtelement (30) eine fluidische Dichtung bildet, wenn sich das mindestens eine Dichtelement (30) im zweiten Kompressionszustand befindet,
c) eine zweite Sperrvorrichtung, die eine Bewegung der Kontaktzone(K) aus dem Dichtungsbereich (40) entgegen der Verschieberichtung (R) in den Führungsbereich (35) verhindert.

2. Ampulle nach Anspruch 1, **dadurch gekennzeichnet, dass** der der Kolben (25) als Zweikomponenten-Spritzgussteil ausgeführt ist und der Grundkörper (45) aus einem thermoplastischen Werkstoff besteht und das mindestens eine Dichtelement (30) aus einem thermoplastischen Elastomer besteht.

3. Ampulle nach einem der vorhergehenden Ansprüche, **dadurch gekennzeichnet, dass** die Ampulle eine erste Sperrvorrichtung umfasst, die ein Entfernen des Kolbens (25) aus dem Ampullenkörper (10) entgegen der Verschieberichtung (R) verhindert.

4. Ampulle nach Anspruch 3, **dadurch gekennzeichnet, dass** die erste Sperrvorrichtung mindestens einen ersten amullenkörperseitigen Anschlag (50, 50'), mindestens einen kolbenseitigen Anschlag (55, 55') und mindestens eine Gleitfläche (60, 60') umfasst.

5. Ampulle nach einem der vorhergehenden Asprüche, **dadurch gekennzeichnet, dass** die zweite Sperrvorrichtung mindestens einen zweiten ampullenk8iperseitigen Anschlag (65, 65'), mindestens einen kolbenseitigen Anschlag (55, 55') und mindestens eine Gleitfläche (60, 60') umfasst.

6. Ampulle nach Anspruch 4 und Anspruch 5, **dadurch gekennzeichnet, dass** der mindestens eine kolbenseitige Anschlag (55, 55') der ersten Sperrvorrichtung identisch ist mit dem mindestens einen kolbenseitigen Anschlag (55, 55') der zweiten Sperrvorrichtung.

7. Ampulle nach Anspruch 6, **dadurch gekennzeichnet, dass** die mindestens eine Gleitfläche (60, 60') der ersten Sperrvorrichtung identisch ist mit der mindestens einen Gleitfläche (60, 60') der zweiten Sperrvorrichtung.

8. Ampulle nach Anspruch 7, **dadurch gekennzeichnet, dass** der Kolben (25) mindestens einen radial federnden Federarm (54, 54') umfasst, welcher in Richtung einer offenen Stirnseite (57) des Ampullenkörpers weist und dass an dem Federarm eine Sperrklinke (52, 52') angeordnet ist, welche den mindestens einen kolbenseitige Anschlag (55, 55') und die mindestens eine Gleitfläche (60, 60') bildet, und der Ampullenkörper (15) mindestens einen ersten Ausschnitt (51, 51') und mindestens einen zweiten zum ersten Ausschnitt in Verschieberichtung (R) versetzten zweiten Ausschnitt (66, 66') aufweist und der erste ampullenkörperseitige Anschlag (50, 50') durch die Wandung des mindestens einen ersten Ausschnitts (51, 51') gebildet wird und der zweite ampullenkörperseitige Anschlag (65, 65') durch die Wandung des mindestens einen zweiten Ausschnitts (66, 66') gebildet wird und die Sperrklinke (52, 52') im entlasteten Zustand des Federarmes (54, 54') in den mindestens einen ersten Ausschnitt (51, 51') sowie den mindestens einen zweiten Ausschnitt (66, 66') hineinragen kann.

9. Ampulle nach Anspruch 8, **dadurch gekennzeichnet**, das die innere Mantelfläche (20) des Ampullenkörpers (10) den mindestens einen Federarm (54, 54') radial so deformieren kann, dass die mindestens eine Sperrklinke (52, 52') federnd gegen die innere Mantelfläche (20) drückt.

10. Ampulle nach einem der vorhergehenden Ansprüche, **dadurch gekennzeichnet, dass** der Innenraum (15) senkrecht zur Verschieberichtung (R) einen von der Kreisform abweichenden Querschnitt aufweist.

11. Ampulle nach einem der vorhergehenden Ansprüche, **dadurch gekennzeichnet, dass** die innere Mantelfläche (20) einen Übergangsbereich (37) aufweist, der den Führungsbereich (35) stetig mit dem Dichtungsbereich (40) verbindet.

12. Ampulle nach einem der vorhergehenden Ansprüche, **dadurch gekennzeichnet, dass** der Ampullenkörper (10) mindestens eine Auslassöffnung (70) aufweist.

13. Ampulle nach einem der vorhergehenden Ansprüche, **dadurch gekennzeichnet, dass** das mindestens eine Dichtelement (30) mindestens zwei Kontaktzonen (K11, K12), (K21, K22) umfasst.

14. Ampulle nach einem der vorhergehenden Ansprüche, **dadurch gekennzeichnet, dass** der Kolben (25) mindestens ein gegenüber dem mindestens einen Dichtelement (30) in Richtung einer Öffnung (57) des Ampullenkörpers versetzt angeordnetes Führungselement umfasst, welches die innere Mantelfläche (20) des Ampullenkörpers (10) kontaktiert.

15. Ampulle nach einem der vorhergehenden Ansprüche, **dadurch gekennzeichnet, dass** der Kolben (25) eine Koppelvorrichtung zur Kopplung mit einem Vorschubantrieb (315) einer Injektions- oder Infusionsvorrichtung (310) umfasst.

16. Ampulle nach einem der vorhergehenden Ansprüche, **dadurch gekennzeichnet, dass** das mindestens eine Dichtelement (30) gemeinsam mit der inneren Mantelfläche (20) des Ampullenkörpers (10) keine Dichtung bezüglich eines Mediums mit sterilisierender Wirkung aufweist, wenn sich das mindestens eine Dichtelement (30) im ersten Kompressionszustand befindet.

17. Ampulle nach einem der vorhergehenden Ansprüche, **dadurch gekennzeichnet, dass** die Ampulle eine Aufziehvorrichtung zur Verschiebung des Kolbens (25) entgegen der Verschieberichtung (R) umfasst.

18. Ampulle für eine fliessfähige Substanz, umfassend mindestens eine erste Teilampulle (100) nach einem der vorhergehenden Ansprüche und eine zweite Teilampulle (200) nach einem der vorhergehenden Ansprüche.

19. Dosiersystem für eine fließfähige Substanz, umfassend:
a) eine Ampulle (300) nach einem der vorhergehenden Ansprüche mit einer Auslassöffnung (70), sowie
b) eine Injektions- oder Infusionsvorrichtung (310), welche mit der Ampulle (300) operativ gekoppelt ist.

## Claims

1. Ampoule for a flowable substance comprising:
a) an ampoule body (10) with an interior space (15) for receiving the flowable substance and with an inner casing surface (20),
b) a plunger (25) comprising at least one sealing element (30) which is arranged in the interior space (15) so that it can be displaced along a displacement direction (R), wherein the at least one sealing element (30) is in contact with the inner casing surface (20) in a contact zone (K) along the outer circumference of the at least one sealing element (30),
wherein the inner casing surface (20) has a guide region (35) and a sealing region (40) along the displacement direction (R) where the cross-section of the interior space (15) in a plane at right angles to the displacement direction (R) in the guide region (35) is designed such that the at least one sealing element (30) has a first compression state when the contact zone (K) is situated in the guide region (35) and in the sealing region (40) is designed such that the at least one sealing element (30) has a second compression state when the contact zone (K) is situated in the sealing region (40) and such that the inner casing surface (20) together with the at least one sealing element (30) forms a fluidic seal when the at least one sealing element (30) is in the second compression state,
c) a second locking device which prevents the contact zone (K) from moving out of the sealing region (40) into the guide region (35) against the displacement direction (R).

2. Ampoule according to claim 1, **characterized in that** the plunger (25) is designed as a two-component injection moulded part and the base body (45) consists of a thermoplastic material and the at least one sealing element (30) consists of a thermosplastic elastomer.

3. Ampoule according to one of the previous claims, **characterized in that** the ampoule has a first locking device which prevents the plunger (25) from being removed from the ampoule body (10) against the displacement direction (R).

4. Ampoule according to claim 3, **characterized in that** the first locking device has at least one first stop (50, 50') on the ampoule body side, at least one stop (55, 55') on the plunger side and at least one sliding surface (60, 60').

5. Ampoule according to one of the previous claims, **characterized in that** the second locking device has at least one stop (65, 65') on the ampoule body side, at least one stop (55, 55') on the plunger side and at least one sliding surface (60, 60').

6. Ampoule according to claim 4 and 5, **characterized in that** the at least one stop (55, 55') on the plunger side of the first locking device is identical to the at least one stop (55, 55') on the plunger side of the second locking device.

7. Ampoule according to claim 6, **characterized in that** the at least one sliding surface (60, 60') of the first locking device is identical to the at least one sliding surface (60, 60') of the second locking device.

8. Ampoule according to claim 7, **characterized in that** the plunger (25) comprises at least one radially resilient spring arm (54, 54') which points towards an open end wall (57) of the ampoule body and that a pawl (52, 52') is arranged on the spring arm said pawl forming the at least one stop (55, 55') on the plunger side and the at least one sliding surface (60, 60'), and the ampoule body (15) has at least one first aperture (51, 51') and at least one second aperture (66, 66') that is displaced relative to the first section in the displacement direction (R) and the first stop (50, 50') on the ampoule body side is formed by the wall of the at least one first aperture (51, 51') and the second stop (65, 65') on the ampoule body side is formed by the wall of the at least one second aperture (66, 66') and the pawl (52, 52') in the relieved state of the spring arm (54, 54') can extend into the at least one first aperture (51, 51') as well as into the at least one second aperture (66, 66').

9. Ampoule according to claim 8, **characterized in that** the inner casing surface (20) of the ampoule body (10) can radially deform the at least one spring arm (54, 54') in such a manner that the at least one pawl (52, 52') is pressed elastically against the inner casing surface (20).

10. Ampoule according to one of the previous claims, **characterized in that** the interior space (15) has a cross-section that deviates from circularity at right angles to the displacement direction (R).

11. Ampoule according to one of the previous claims, **characterized in that** the inner casing surface (20) has a transition region (37) which permanently joins the guide region (35) with the sealing region (40).

12. Ampoule according to one of the previous claims, **characterized in that** the ampoule body (10) has at least one outlet opening (70).

13. Ampoule according to one of the previous claims, **characterized in that** the at least one sealing element (30) comprises at least two contact zones (K11, K12), (K21, K22).

14. Ampoule according to one of the previous claims, **characterized in that** the plunger (25) comprises at least one guide element that is displaced relative to the at least one sealing element (30) in the direction of an opening (57) of the ampoule body and said guide element contacts the inner casing surface (20) of the ampoule body (10).

15. Ampoule according to one of the previous claims, **characterized in that** the plunger (25) comprises a coupling device for coupling it to a propulsion drive (315) of an injection or infusion device (310).

16. Ampoule according to one of the previous claims, **characterized in that** the at least one sealing element (30) together with the inner casing surface (20) of the ampoule body (10) does not form a seal with regard to a medium having a sterilizing effect when the at least one sealing element (30) is in the first compression state.

17. Ampoule according to one of the previous claims, **characterized in that** the ampoule comprises a pulling device to displace the plunger (25) against the displacement direction (R).

18. Ampoule for a flowable substance comprising at least a first ampoule part (100) according to one of the previous claims and a second ampoule part (200) according to one of the previous claims.

19. Metering system for a flowable substance comprising:
a) an ampoule (300) according to one of the previous claims with an outlet opening (70), as well as
b) an injection or infusion device (310) which is operatively coupled to the ampoule (300).

## Revendications

1. Ampoule pour une substance coulante, comprenant :
a) un corps d'ampoule (10) avec un espace intérieur (15) pour recevoir la substance coulante et avec une surface enveloppante intérieure (20),
b) un piston (25) avec au moins un élément d'étanchéité (30) qui est situé de manière déplaçable dans un sens de déplacement (R) dans l'espace intérieur (15), l'au moins un élément d'étanchéité (30) étant en contact avec la surface enveloppante intérieure (20) dans une zone de contact (K) le long du pourtour extérieur de l'au moins un élément d'étanchéité (30),
la surface enveloppante intérieure (20) présentant, dans le sens du déplacement (R), une zone de guidage (35) et une zone d'étanchéité (40), la section de l'espace intérieur (15) étant réalisée de manière telle, dans un plan transversalement par rapport au sens du déplacement (R) dans la zone de guidage (35), que l'au moins un élément d'étanchéité (30) présente un premier état de compression lorsque la zone de contact (K) se trouve dans la zone de guidage (35), et étant réalisée de manière telle, dans la zone d'étanchéité (40), que l'au moins un élément d'étanchéité (30) présente un deuxième état de compression lorsque la zone de contact (K) se trouve dans la zone d'étanchéité (40), et de manière telle que la surface enveloppante intérieure (20) constitue, conjointement avec l'au moins un élément d'étanchéité (30), une étanchéité aux fluides lorsque l'au moins un élément d'étanchéité (30) se trouve dans le deuxième état de compression,
c) un deuxième dispositif de blocage qui empêche un mouvement de la zone de contact (K) allant de la zone d'étanchéité (40) vers la zone de guidage (35) à l'encontre du sens du déplacement (R).

2. Ampoule selon la revendication 1, **caractérisé en ce que** le piston (25) se présente sous la forme d'une pièce bicomposite moulée par injection et le corps de base (45) se compose d'un matériau thermoplastique et l'au moins un élément d'étanchéité (30) se compose d'un élastomère thermoplastique.

3. Ampoule selon l'une des revendications précédentes, **caractérisée en ce que** l'ampoule comprend un premier dispositif de blocage qui empêche un retrait du piston (25) hors du corps d'ampoule (10) à l'encontre du sens du déplacement (R).

4. Ampoule selon la revendication 3, **caractérisée en ce que** le premier dispositif de blocage comprend au moins une première butée (50, 50') située du côté du corps d'ampoule, au moins une butée (55, 55') située du côté du piston et au moins une surface de glissement (60, 60').

5. Ampoule selon l'une des revendications précédentes, **caractérisée en ce que** le deuxième dispositif de blocage comprend au moins une deuxième butée (65, 65') située du côté du corps d'ampoule, au moins une butée (55, 55') située du côté du piston et au moins une surface de glissement (60, 60').

6. Ampoule selon la revendication 4 et selon la revendication 5, **caractérisée en ce que** l'au moins une butée (55, 55') du premier dispositif de blocage située du côté du piston est identique à l'au moins une butée (55, 55') du deuxième dispositif de blocage située du côté du piston.

7. Ampoule selon la revendication 6, **caractérisée en ce que** l'au moins une surface de glissement (60, 60') du premier dispositif de blocage est identique à l'au moins une surface de glissement (60, 60') du deuxième dispositif de blocage.

8. Ampoule selon la revendication 7, **caractérisée en ce que** le piston (25) comprend au moins un bras élastique (54, 54') à élasticité radiale qui est pointé en direction d'une face frontale ouverte (57) du corps d'ampoule et **en ce qu'**est situé, sur le bras élastique, un cliquet d'arrêt (52, 52') qui constitue l'au moins une butée (55, 55') située du côté du piston et l'au moins une surface de glissement (60, 60'), et le corps d'ampoule (15) comporte au moins une première échancrure (51, 51') et au moins une deuxième échancrure (66, 66') décalée par rapport à la première échancrure dans le sens du déplacement (R), et la première butée (50, 50') située du côté du corps d'ampoule est constituée par la paroi de l'au moins une première échancrure (51, 51'), et la deuxième butée (65, 65') située du côté du corps d'ampoule est constituée par la paroi de l'au moins une deuxième échancrure (66, 66'), et le cliquet d'arrêt (52, 52'), à l'état détendu du bras élastique (54, 54'), peut faire saillie dans l'au moins une première échancrure (51, 51') ainsi que l'au moins une deuxième échancrure (66, 66').

9. Ampoule selon la revendication 8, **caractérisée en ce que** la surface enveloppante intérieure (20) du corps d'ampoule (10) peut déformer l'au moins un bras élastique (54, 54') radialement de manière telle que l'au moins un cliquet d'arrêt (52, 52') pousse élastiquement contre la surface enveloppante intérieure (20).

10. Ampoule selon l'une des revendications précédentes, **caractérisée en ce que** l'espace intérieur (15) présente, perpendiculairement par rapport au sens du déplacement (R), une section différente de la forme circulaire.

11. Ampoule selon l'une des revendications précédentes, **caractérisée en ce que** la surface enveloppante intérieure (20) présente une zone de transition (37) qui relie en continu la zone de guidage (35) et la zone d'étanchéité (40).

12. Ampoule selon l'une des revendications précédentes, **caractérisée en ce que** le corps d'ampoule (10) présente au moins un orifice de sortie (70).

13. Ampoule selon l'une des revendications précédentes, **caractérisée en ce que** l'au moins un élément d'étanchéité (30) comporte au moins deux zones de contact (K11, K12), (K21, K22).

14. Ampoule selon l'une des revendications précédentes, **caractérisée en ce que** le piston (25) comprend au moins un élément de guidage situé de manière décalée par rapport à l'au moins un élément d'étanchéité (30) dans le sens d'une ouverture (57) du corps d'ampoule, lequel élément de guidage contacte la surface enveloppante intérieure (20) du corps d'ampoule (10).

15. Ampoule selon l'une des revendications précédentes, **caractérisée en ce que** le piston (25) comprend un dispositif de couplage pour couplage avec un entraînement d'avance (315) d'un dispositif d'injection ou de perfusion (310).

16. Ampoule selon l'une des revendications précédentes, **caractérisée en ce que** l'au moins un élément d'étanchéité (30), conjointement avec la surface enveloppante intérieure (20) du corps d'ampoule (10), ne présente pas d'étanchéité par rapport à un milieu à effet stérilisant lorsque l'au moins un élément d'étanchéité (30) se trouve dans le premier état de compression.

17. Ampoule selon l'une des revendications précédentes, **caractérisée en ce que** l'ampoule comprend un dispositif de remontage pour déplacer le piston (25) à l'encontre du sens du déplacement (R).

18. Ampoule pour une substance coulante, comprenant au moins une première ampoule partielle (100) selon l'une des revendications précédentes et une deuxième ampoule partielle (200) selon l'une des revendications précédentes.

19. Système de dosage pour une substance coulante, comprenant :
a) une ampoule (300) selon l'une des revendications précédentes avec un orifice de sortie (70), ainsi que
b) un dispositif d'injection ou de perfusion (310) qui est couplé opérationnellement à l'ampoule (300).
